(19) **Europäisches Patentamt**
**European Patent Office**
**Office européen des brevets**

(11) **EP 3 396 946 A1**

(12) **EUROPEAN PATENT APPLICATION**

(43) Date of publication:
**31.10.2018 Bulletin 2018/44**

(51) Int Cl.:
*H04N 7/18* (2006.01)          *G06K 9/00* (2006.01)
*A61B 5/00* (2006.01)

(21) Application number: **17167955.8**

(22) Date of filing: **25.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **Long, Xi**
  **5656 AE Eindhoven (NL)**
• **Ten Hoor, Lauren**
  **5656 AE Eindhoven (NL)**

• **van der Sanden, Eric**
  **5656 AE Eindhoven (NL)**
• **den Boer, Sebastiaan**
  **5656 AE Eindhoven (NL)**
• **Gelissen, Jos**
  **5656 AE Eindhoven (NL)**
• **Prevoo, Yves**
  **5656 AE Eindhoven (NL)**
• **Zwartkruis-Pelgrim, Elly**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **SYSTEM, METHOD AND COMPUTER PROGRAM FOR MONITORING A BABY**

(57)     The invention relates to a system for monitoring a baby (7), comprising a motion data providing unit (10) for providing motion data (12, 14) indicative of motions within a stream of video images (200), a region of interest providing unit (20) for providing a region of interest (22) in the video images (200), a classifying unit (30) for classifying the provided motion data (12, 14) into at least motion data (12, 14) from inside the region of interest (22, 28) and from outside the region of interest (22, 28), an event detection unit (40) for detecting an event by evaluating motion data (12, 14) from inside the region of interest (22, 28) and motion data (12, 14) from outside the region of interest (22, 28). The system and corresponding method and corresponding computer program allow for a more reliable determination of baby in or out of bed status.

FIG. 1

**Description**

FIELD OF THE INVENTION

The invention relates to a system, method and corresponding computer program for monitoring a baby.

BACKGROUND OF THE INVENTION

It has long been a desire for parents and healthcare professionals to track the sleep of babies to, among others, monitor and evaluate the babies' mental and physical development through assessing the babies' sleep quality. The assessment of baby sleep quality is often done by analyzing several sleep statistics including sleep efficiency, total sleep time, number of awakenings, sleep onset latency, i.e. a duration between the time at which a baby is put to sleep until it eventually falls asleep, and sleep stages such as active wake, quiet wake, active sleep and quiet sleep.

A wearable motion sensor is usually not acceptable for babies because their skin is very sensitive. Therefore, there are currently a lot of camera-based baby monitors available due to their primary advantage of unobtrusiveness of monitoring.

WO 2015/091582 A1 discloses a baby monitoring system which gives insight in the sleeping behavior of a child based on the motion of the child in the bed. The baby monitoring system comprises a video camera, a motion estimator and a processor to classify the observed motions into events. A set of events gives a parent an insight in the sleeping behavior of the child. The observed motions are classified into small amplitude motions, intermediate amplitude motions and large amplitude motions. Along with a sound sensor, the motions and in particular the amplitude of the motions are used to determine baby in or out of bed status and events including "baby is put to bed" and "baby is taken out of bed".

While it is crucial to know whether the baby is in bed/crib or out of bed/crib, the known solutions fail to reliably determine the baby in or out of bed status since the observed motions can be caused by a baby's body movements, parent activity, other disturbances, etc. and the assessment substantially depends on the placement of the camera with respect to the bed.

SUMMARY OF THE INVENTION

It has therefore been an object of the present invention to provide a system, method and computer program for monitoring a baby which allows for a more reliable determination of baby in or out of bed status.

In a first aspect, a system for monitoring a baby is provided. The system comprises a motion data providing unit for providing motion data indicative of motions within a stream of video images, a region of interest providing unit for providing a region of interest in the video images, a classifying unit for classifying the provided motion data into at least motion data from inside the region of interest and from outside the region of interest, an event detection unit for detecting an event by evaluating motion data from inside the region of interest and motion data from outside the region of interest.

Since the event detection unit is configured to detect an event based on an evaluation of motion data from both inside the region of interest and motion data from outside the region of interest, motion which occurs outside the region of interest, i.e. which preferably is not attributable to a baby, can be distinguished from and evaluated differently from motion data from inside the region of interest, which can for instance more likely correspond to motion of the baby.

Preferably, the stream of video images comprises images suitable for monitoring the baby, wherein the evaluation of motion data of the stream of video images allows for a simpler and at the same tome more reliable evaluation as compared to, for instance, a recognition algorithm for analyzing the image content of the video images.

Advantageously, based on the more reliably detected events, the system according to the invention can be suitable for more reliably determining whether the baby is in or out of bed.

Since the event detection unit considers the classification of the classifying unit, the classification of the motion data into motion data from inside the region of interest and from outside the region of interest advantageously allows a more subtle detection of events and thereby increases the reliability of the system.

Preferably, motion data indicative of motions within a stream of video images comprises a measure of accumulated differences between two consecutive video images, wherein also other measures for motion data are contemplated. In one example, the more pixels or image regions change between two consecutive video images in the stream of video images, the higher the values of corresponding motion data will be.

The motion data providing unit can be a motion data determination unit for determining motion data based on a received stream of video images, for instance from a received video recording or from a live stream of video images received from a camera device. In a different embodiment, the motion data providing unit can comprise a motion data storing unit for storing motion data previously calculated in connection to a recorded stream of video images. In this embodiment, the motion data providing unit can then provide the stored motion data from the motion data storing unit.

In an embodiment at least one of the motion data from inside the region of interest and the motion data from outside the region of interest is provided as the sum of motion over a period of time of the stream of video images.

Since preferably the motion data providing unit is configured to provide the motion data as the sum of motion over a period of time of the stream of video images, the complexity of computation and thus the computational effort for monitoring the baby can be further reduced. Expressed differently, the stream of video images is generally comprised of images with a high sampling frequency exceeding 10 frames per second. In contrast thereto, in particular an estimation whether a baby is in bed or not has a significantly lower frequency of changes. Therefore, in some embodiments periods of time, to which it is referred to as epochs, are employed for summing the motion data to reduce the sampling frequency of the motion data.

Preferably, the duration of an epoch is 30s, while also longer or shorter epochs are contemplated in other embodiments.

Further preferably, the epochs are non-overlapping in time and completely divide the stream of video images into a plurality of epochs. Preferably, motion data within the region of interest and outside the region of interest is indicative of an amplitude of motions within and outside the region of interest, respectively, during the corresponding period of time.

In an embodiment the motion data providing unit is configured to provide the motion data for a stream of video images which comprises several of the periods of time, i.e. the epochs.

Advantageously, since the stream of video images comprises several of the periods of time, a sufficient statistical significance for reliably monitoring the baby can be achieved. In one embodiment, the stream of video images comprises a duration of 24 hours, wherein also shorter or longer durations of the stream of video images can be employed. However, in case the video images are provided in the form of recorded video images, in one embodiment it is preferred that the stream of recorded video images be analyzed and evaluated in chunks of about 24 hours due to the informative value of a evaluation of the monitoring on a daily basis.

In an embodiment the event detection unit is configured to detect at least one of i) baby put to bed and ii) baby taken out of bed as an event.

Since the event detection unit is configured to detect whether the baby is put to bed or the baby is taken out of bed as an event, it can be determined with increased accuracy whether the status of baby is or out of bed changes, i.e. a transition between both statuses occurs, at a certain point in time. Preferably, it can be determined that the baby is in bed after an event identified as baby put to bed is detected by the event detection unit and, similarly, it is determined that the baby is out of bed after an event classified as baby taken out of bed has been detected. In other embodiments, also alternative or additional events can be detected by the event detection unit.

In an embodiment the system further comprises a filter unit for filtering motion data from inside the region of interest and from outside the region of interest.

Since the event detection unit detects events based on motion data, and since a filter unit configured to filter motion data is provided, the detection by the event detection unit can be more reliably since the underlying data is improved through the filtering. Preferably, the filtering can include comparing a magnitude of the motion data with a threshold, for instance, such as to discard motion data from inside the region of interest and/or from outside the region of interest which does not exceed a predetermined threshold. Thereby, motion most probably being accountable to noise or the like will not accidentally be detected as an event by the event detection unit.

In an embodiment the event detection unit is configured to detect an event if i) motions inside the region of interest and motions outside the region of interest are larger than zero and ii) a ratio between motions inside the region of interest and motions outside the region of interest is below a threshold.

Particularly in case in which the event is one of whether the baby is put to bed or taken out of bed it can be assumed that motion outside the region of interest is not too small compared to motion inside the region of interest. Expressed differently, in case the ratio would become too large, it is likely that motions only originate from the baby and no parent or other caregiver putting the baby in bed or taking the baby out of bed is involved.

In an embodiment the system further comprises a baby in bed determination unit for determining whether the baby is in bed or not based on events detected by the event detection unit and based on motion data from inside the region of interest.

Preferably, the baby in bed determination unit is configured to determine, based on the events acting as possible transitions between in and out of bed status and based on motion data from inside the region of interest whether the baby is in bed or not. In this embodiment it is assumed that motion from outside the region of interest is irrelevant to the baby in bed status between events. However, in other embodiments, of course additional data can be considered.

In an embodiment the baby in bed determination unit is configured to compare an amplitude of motion data from inside the region of interest with a predetermined threshold and to determine the baby as out of bed for the entire time of the stream of video images in case the amplitude does not exceed the predetermined threshold.

Despite events being detected throughout the stream of video images, the baby can nevertheless not be in bed at any time. Therefore, in this embodiment, an amplitude of the motion data inside the region of interest is compared to a predetermined threshold and in case no amplitude exceeds this predetermined threshold, i.e. a maximum amplitude of the motion data from inside the region of interest for the entire stream of video images lies below the threshold, the baby in bed determination unit determines the baby as out of bed for the entire time of the stream of video images.

Preferably, the entire time of the stream of video images corresponds to an entire recoding, which can correspond to a period of, for instance, 24 hours. However, motion data corresponding to a longer or shorter period of the stream of video images can of course also be evaluated in other embodiments.

In an embodiment the baby in bed determination unit is configured to define a period between two events and to analyze the percentage of time with motion during the period for determining whether the baby is in bed or not.

Preferably, the percentage of time is calculated based on epochs of motion data. Advantageously, epochs of motion data, in which the motions exceed a predefined threshold, are considered as "epochs with motion", and a number of the epochs with motion is compared to the overall number of epochs between the two events.

In an embodiment the baby in bed determination unit is configured to discard a predetermined percentage of the period between two events from the motion data, wherein the motion data is discarded at the beginning and the end of the period.

In this embodiment, motion data at the beginning and the end of the period between two events, which is most likely caused by motion of the parent or caregiver, i.e. motion outside the region of interest, is not taken into account for determining whether the baby is in bed or not. More precisely, since the beginning and the end of the period most likely comprises motion, which does not originate from the baby and thus could deteriorate the accuracy of determining whether the baby is in bed or not, it is advantageous for this motion data to be discarded.

In an embodiment the baby in bed determination unit is configured to determine the baby as in bed for the period in case the percentage of time with motion exceeds a predetermined threshold.

A high percentage of time with motion between two events is preferably indicative of the baby being in bed during the period between two events. To the contrary, in case of a low percentage, likelihood for the baby being out of bed is high. In an embodiment the system further comprises a smoothing unit for smoothing the determination result of the baby in bed determination unit.

The frequency of events and thus the frequency of periods, during which the baby can be in bed or out of bed, based on the determination of the baby in bed determination unit, can be comparably high, which does not reflect a heuristically discernible situation, in which a baby is put to bed and/or taken out of bed only several times per day. Accordingly, the smoothing unit preferably accounts for this heuristically determined fact and preferably smoothes out very short periods of the baby determined in bed/out of bed. For instance, the smoothing unit can comprise a filter unit such as a median filter with a sliding window that can be used after the determination by baby in bed determination unit to filter out very short periods of baby in bed/baby out of bed. However, of course also other aspects of smoothing the baby in bed determination result are contemplated.

In an embodiment the region of interest providing unit is configured to provide a customized region of interest based on the video images, wherein the customization is either determined automatically based on previous video images or manually selected by a user.

The customized region of interest can accurately reflect the region, in which the baby is most likely to be as seen in the stream of video images. Accordingly, motion data from within the region of interest more likely can be associated with the baby, while motion from outside the region of interest more likely corresponds to motion not being accountable to the baby. Preferably, the customized region of interest can be defined, e.g. manually selected, by a user using a user interface. The manual selection by the user can either be constant or also change over time. Additionally or alternatively, an automatically determination based on previous video images of the stream of video images can be carried out to provide the customized region of interest directly or even as an adjustment to the region of interest manually selected by the user.

In an alternative embodiment, the region of interest is provided as a fixed region of interest corresponding to a region of the video image which is predefined, e.g. a rectangular region of interest in the center of the video image having predefined margins to the different edges of the video image. For instance, the region of interest can correspond to approximately ninety percent of the area of the video images, while the shape of the region of interest corresponds to the shape of the video images with the region interest centered therein. However, in other embodiments, neither the shape of the fixed region of interest has to correspond to the shape of the video images, e.g. the shape can deviate from a rectangular form, nor does the fixed region of interest have to be centered within the video images.

In an embodiment the region of interest providing unit is configured to provide a region comprising a bed of the baby as the region of interest.

Since the region of interest comprises a bed of the baby, the region of interest comprises the region in which detectable motions are most likely indicative of whether the baby is in bed or not. Preferably, the region of interest is provided as a customized region of interest, wherein an automatically determination of the customized region of interest can be determined by using a bed detection algorithm for automatically detecting the bed of the baby based on the video images.

Advantageously, the bed detection algorithm eliminates the need of adapting the customized region of interest in case a place or angle at which or under which the video images are recorded is changed over time.

In an embodiment the region of interest providing unit is configured to detect a misalignment of the region of interest and to at least one of i) notify about a detected misalignment and ii) correct the region of interest. Misalignment of the region of interest, e.g. the region of interest does not cover a bed of the baby or the bed is not fully visible, can cause incorrect determination of whether the baby is in bed or not. By notifying and/or correcting a misaligned region of interest, reliability of the determination whether the baby is in bed or not is improved.

In an embodiment the region of interest providing unit is configured to detect the misalignment of the region of interest based on motion of a camera which records the stream of video images or a change of view of the camera based on at least one of the provided motion data and the video images.

In an embodiment the region of interest providing unit is configured to determine motion of the camera or a change of view of the camera by at least one of i) analyzing the uniformity of motion over the video images and ii) determining and monitoring outlines of a bed of the baby in the video images.

Advantageously, by analyzing the uniformity of motion, movement of the camera can be detected in case the motion is uniform, i.e. all pixels substantially move in the same direction. The outlines of a bed of the baby are preferably determined upon installation of the system according to the invention, when there is no motion detected in the video stream. The outlines determined at the setup of the system can be taken as a reference such that deviations from this reference outlines can be indicative of a misalignment of the region of interest. Preferably, the outlines are determined in a view of the image different from a standard RGB view in which the images was recorded, for instance in contrast view. In contrast view, outlines like the one of the bed of the baby are highlighted and can be determined more efficiently and accurately.

Preferably, a threshold for determining motion of the camera and/or a change of view, above which the region of interest is considered misaligned, is provided.

In an embodiment the region of interest providing unit is configured to detect a misalignment of the region of interest based on data from a sensor component, wherein the sensor component includes at least one of an accelerometer, a gyroscope and a proximity sensor. The sensor component can, for instance, be integrated in the camera which records the stream of video images. Additionally or alternatively, the sensor component can also be attached to a bed of the baby such that motion of the bed of the baby can be determined. A change on the planar axes or in the tilt angle of the accelerometer indicates a change in the view of the camera, in case it is attached to the camera. The gyroscope likewise allows detecting a change of the viewing angle of the camera. Finally, the proximity sensor allows detecting both a change of position of the sensor itself, i.e. the camera it is attached to, relative to the room and a change of items or objects in the room, e.g. the bed of the baby.

In an embodiment the region of interest providing unit is configured to detect a misalignment of the region of interest based on one or more markers, preferably infrared reflective markers, attached to an object within the region of interest. The markers are attached at a known position of the object, e.g. the bed of the baby, and allow a determination of the object based on the position of the marker which is visible in the video stream and/or detectable by the camera. Infrared reflective markers, which are not visually distracting to humans, are preferred, while any suitable marker as known in the art is contemplated.

In a further aspect a method of monitoring a baby is provided. The method comprises providing motion data indicative of motions within a stream of video images, providing a region of interest in the video images, classifying the provided motion data into at least motion data from inside the region of interest and from outside the region of interest, and detecting an event by evaluating motion data from inside the region of interest and motion data from outside the region of interest.

In a further aspect a computer program for monitoring a baby is provided, the computer program comprising program code means for causing a system for monitoring a baby according to the invention to carry out the method of monitoring a baby according to the invention, when the computer program is run on the system.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a system for monitoring a baby according to the invention,
Fig. 2A shows schematically and exemplarily excerpts from a 24 hour video recording,
Figs. 2B and 2C show schematically and exemplarily motion data determined from the recording shown in Fig. 2A,
Figs. 3A and 3B show schematically and exemplarily a video image with a region of interest,

Fig. 4 shows schematically and exemplarily the output of various units of the system for monitoring a baby,
Fig. 5 shows schematically and exemplarily a flowchart of a baby in/out of bed detection method,
Fig. 6 shows schematically and exemplarily a further flowchart of a method for monitoring a baby,
Figs. 7A and 7B show schematically and exemplarily motion patterns for determining misalignment of the region of interest,
Figs. 8A and 8B schematically and exemplarily illustrate two camera views before and after motion of the bed of the baby,
Figs. 9A and 9B schematically and exemplarily illustrate two arrangements of markers, and
Figs. 10A to 10C schematically and exemplarily illustrate embodiments and an effect of markers.

DETAILED DESCRIPTION OF EMBODIMENTS

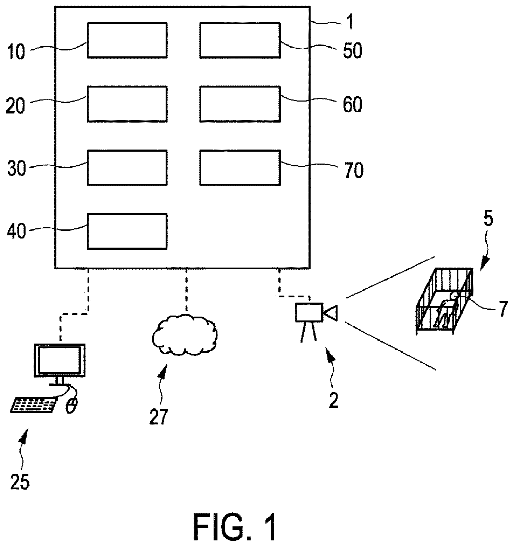

Fig. 1 schematically and exemplarily illustrates a system 1 for monitoring a baby. System 1 comprises a motion data providing unit 10, a region of interest providing unit 20, a classifying unit 30, an event detection unit 40, a filter unit 50, a baby in bed determination unit 60 and a smoothing unit 70. While system 1 in this example comprises all the units mentioned before, it should be noted that particularly the filter unit 50, baby in bed determination unit 60 and smoothing unit 70 are optional units and do not have to be implemented in all systems according to the invention.

System 1 and all the units comprised therein can be realized in the form of dedicated hardware and/or software to be executed on any form of suitable computer apparatus, including general purpose computers, smart phones, tablets or servers. It should be noted that one, more or all of the motion data providing unit 10, the region of interest providing unit 20, the classifying unit 30, the event detection unit 40, the filter unit 50, the baby in bed determination unit 60 and the smoothing unit 70 can be provided at a different physical location from the rest of system 1, such as being implemented as a web service, a standalone module, and the like.

In this example, system 1 is configured to communicate with a camera 2, which is configured to record a bed 5, in which a baby 7 is located or not. System 1 and the dedicated units provided therein then allow for a reliable determination whether baby 7 is in bed 5 or not.

Further, system 1 provides an optional user interface means 25, for instance, a general purpose computer or a smart phone, a web interface and the like. Using user interface means 25, data can be input into system 1, or any of the units thereof, and/or output from system 1 to the user. For instance, an indication of the evaluation whether baby 7 is in bed 5 or not can be output to the user.

Finally, system 1 can provide a communication link to at least one further entity 27, such as the Internet, including web servers, cloud storage, and the like. One, more or all of the functions of system 1 including data storage can be outsourced to the further entity 27. Variations to this concept are obvious implementations to those skilled in the art.

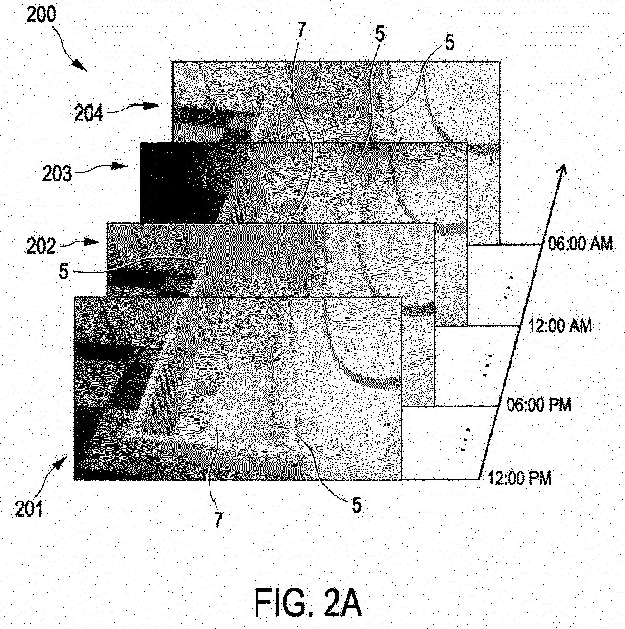

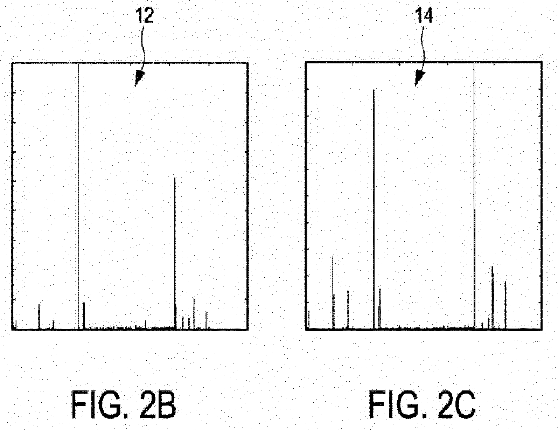

Motion data providing unit 10 is configured to provide motion data, which is indicative of motions within a stream of video images 200. The stream of video images 200, as illustrated in Fig. 2, can be received from camera 2 or can correspond to a previously recorded image stream, which can exemplarily be stored by the further entity 27. As an example, motion data providing unit 10 can implement the method disclosed in WO 2015/091582. In this example, since the stream of video images is provided at a sampling frequency of larger than 10 Hz, it is useful to reduce the calculation complexity through converting the motion data to continuous non-overlapping segments, called epochs, having an appropriate length. A sampling frequency of several Hz is too high for determining whether baby 7 is in bed 5 or not. For example, the motion data can be converted into non-overlapping 30s epochs by computing a sum of the raw data for each epoch. It should be noted that this length is the standard for sleep scoring according to the guideline from the American Academy of Sleep Medicine (AASM) to fit the further sleep stage analysis, while the length and form of computation can of course be changed depending on the particular application.

Fig. 2 schematically and exemplarily illustrates a 24 hours recorded stream of video images 200. In Fig. 2A, four exemplary video images 201, 202, 203 and 204 out of the 24 hrs recording are illustrated. In this example, video image 201 is taken at 12 pm, video image 202 at 6 pm, video image 203 at 12 am and video image 204 at 6 am, i.e. each being taken with a time difference of 6 hrs. It can be seen that bed 5 is visible in all video images 201, 202, 203 and 204, while baby 7 is in bed 5 only in video images 201 and 203. Expressed differently, baby 7 is in bed 5 at the time of video images 201 and 203, while it is out of bed at the time of video images 202 and 204.

For the recording illustrated in Fig. 2A, Figs. 2B and 2C illustrate corresponding motion data 12 and 14, wherein a time is illustrated on a horizontal axis and an amplitude of motions illustrated on a vertical axis. While motion data illustrated in Fig. 2B corresponds to the motion data at the original sampling rate of the video images, for instance 15 Hz, motion data 14 illustrated in Fig. 2C corresponds to the motion data after the data has been converted to non-overlapping segments, called epochs, of 30s duration each. In this example, the number of consecutive motion data values to be considered is reduced by a factor 450 compared to the original number of video images.

Region of interest providing unit 20 is configured to provide a region of interest in the video images. Whether baby 7 is in or out of bed 5 is to be identified based on the recorded motion data 12 or 14. However, the total amounts of motions from each video image frame is a combination of motions possibly caused by baby 7, for instance baby body movements, parent activity such as putting baby in bed or taking baby out of bed, parent visit and other disturbances. Therefore, region of interest providing unit 20 provides a region of interest, which is set to a region of the video frames in which the motions most likely are attributable to baby 7. More general, the region of interest is set to try to specify motions from different subjects/objects caused by different activities/behaviors. Therefore, the region of interest can help in characterizing and separating the activities and behaviors and therefore increase the accuracy of a detection whether the baby 7 is in or out of bed 5.

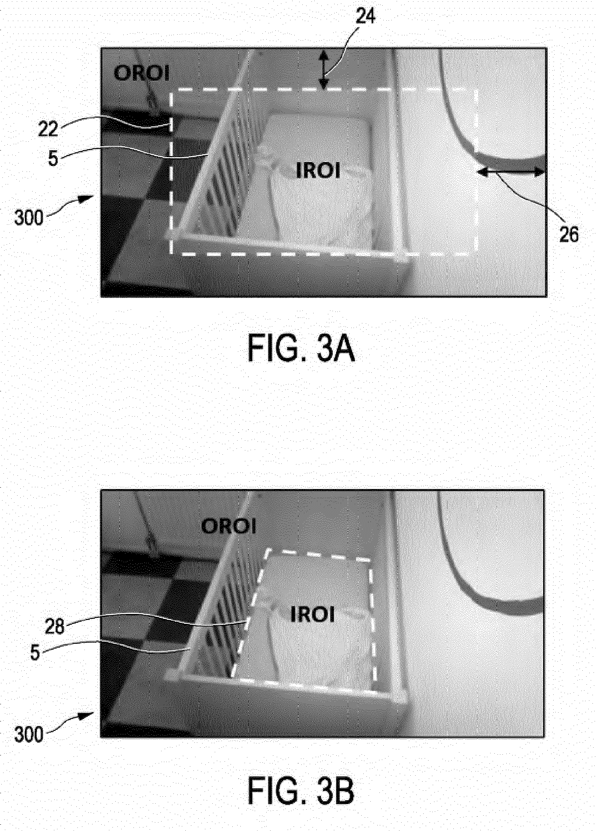

Fig. 3A schematically and exemplarily illustrates a video image 300, in which a region of interest 22 is defined. In this example, region of interest 22 is a fixed region of interest having a distance 24 from the upper and/or lower edge of video image 300 and a distance 26 from a left and/or right edge of video image 300. Region of interest 22 is centered within video image 300 and covers most of the region, in which a bed 5 is located. In this example, region of interest 22 is fixed and, for instance, be set through a factory setting of region of interest providing unit 20. In other examples of fixed regions of interest, the region of interest does not have to be centered within video image 300 and can be of arbitrary shape.

Fig. 3B schematically and exemplarily illustrates a different region of interest 28 for the same video image 300 as shown in Fig. 3A. Region of interest 28 is a customized region of interest adapted specifically to the shape of bed 5. Customized region of interest 28 can be defined through a manual input of the user or automatically defined, for instance by bed frame detection algorithms based on video frames, such as based on video image 300.

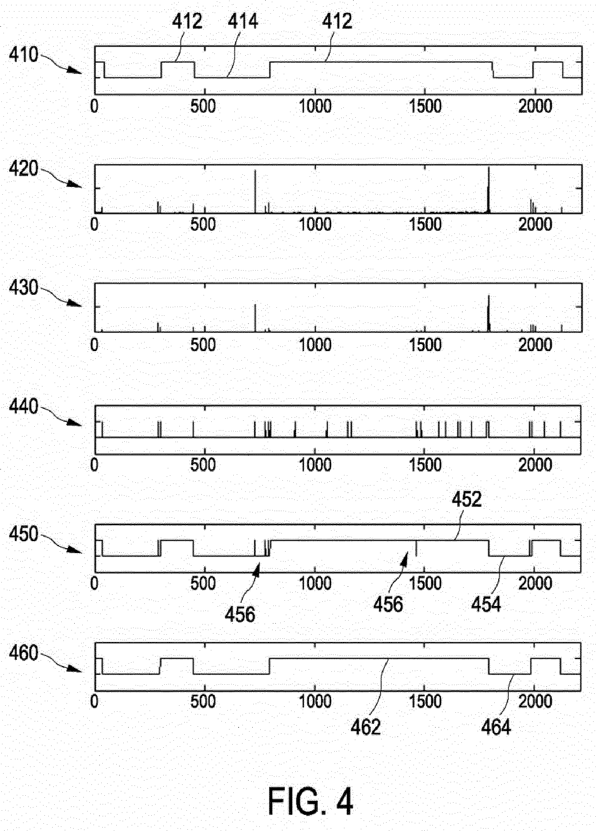

While it can be assumed that customized regions of interest 28 can provide a better discrimination between motions from baby 7 when in bed 5 and those from other subjects/objects when compared to the fixed region of interest 22. However, a fixed region of interest 22 provides the advantage that it is generically valid and does not have to be adapted in case the camera, e.g. camera 2, changes place or angle during recording. Further, no input by a user or no dedicated algorithm is needed for determining the fixed region of interest 22. For one example recording, a difference between fixed region of interest 22 and customized region of interest 28 will be discussed with reference to Fig. 4.

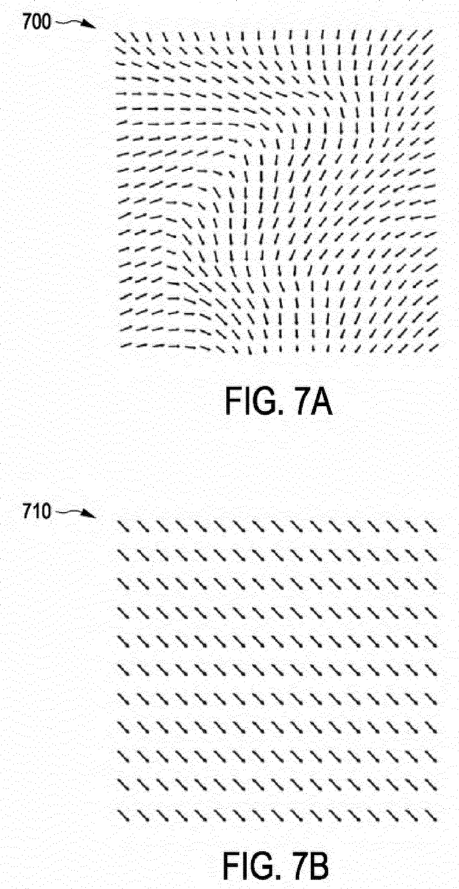

The region of interest is generally basically outlined by the sides or edges of bed 5. Region of interest providing unit 20 is preferentially configured to determine whether the region of interest 22, 28 is misaligned, i.e. deviates from, for instance, the area of bed 5 more than a predetermined threshold. The determination of a misalignment of the region of interest 22, 28 will be discussed with reference to Figs. 7 to 10 below.

Classifying unit 30 is configured to classify the provided motion data 12, 14 into at least motion data from within the region of interest 22, 28 and from outside the region of interest. While in one example motion data is provided from the stream of video images first and then classified into motion from inside the region of interest and outside the region of interest, in different examples also the video images themselves can directly be classified into video data from inside the region of interest and outside the region of interest, wherein the motion data is then provided directly from the already classified video image data. Variations to this concept are obvious to persons skilled in the art. It should further be noted that while in the examples one region of interest 22, 28 is provided, in further examples also additional regions of interest, such as for situations in which more than one bed is visible in a camera image, can of course be contemplated. The inventive concept of the invention can be extended to as many regions of interest as desired.

Event detection unit 40 is configured to detect an event by evaluating motion data 12, 14 from inside the region of interest 22, 28 and from outside the region of interest. In this example, events correspond to determined activities carried out by parent, namely i) baby 7 is put to bed 5 and ii) baby 7 is taken out of bed 5. The events are in this example considered candidates for the transitions between baby in bed and baby out of bed status. Event detection unit 40 is in this example configured to determine events based on a set of rules. Motions (mot) based on motion data 12 or 14 have been classified into motion within the region of interest (mot_iroi) and motion outside the region of interest (mot_oroi).

Motions, which are less than a certain percentile throughout the entire recording, for instance the exemplarily 24 hrs illustrated with reference to Fig. 2, in the region of interest or outside the region of interest are not events, i.e. they are filtered out by setting the motions to zero, for instance by filter unit 50. Relatively small amplitude motions are often caused by movements of the body of baby 7 or other disturbances instead of a transition, i.e. one of the two parent activities, which can change the status of the baby from in bed to out of bed and vice versa.

Accordingly, filter unit 50 can in this example filter out periods of motion data, e.g. epochs, which have a maximum motion amplitude that is too low for any parent or caregiver involvement to be likely during the corresponding period, wherein this parent or caregiver involvement would however be a prerequisite for a baby put to bed/baby taken out of bed event.

Both motions within and outside the region of interest 22, 28 should be larger than zero, i.e. also not be filtered out in the first place, which means that the motions should be relatively large, since activities should be happening both inside and outside the region of interest when a parent is putting baby 7 to bed 5 or taking baby 7 out of bed 5, respectively.

Finally, a ratio between the motion within the region of interest 22, 28 and the motion outside the region of interest should be less than a certain value, called r. This is, in this example, because it is expected that motion outside the region of interest 22, 28 should be not too small compared with motion inside the region of interest 22, 28, when the parent is putting baby 7 in bed 5 or taking baby 7 out of bed 5, otherwise it could possibly be the case that only large movements of the body of baby 7 would be determined as an event.

The rules can be summarized in the following equations:

$$mot\_iroi = 0, mot\_oroi = 0, \quad if \ mot\_iroi < prct\_iroi \ AND \ mot\_oroi < prct\_oroi \quad (1)$$

$$EVENT = 1, \quad if \ mot\_iroi > 0 \ AND \ mot\_oroi > 0 \ AND \ mot\_iroi / mot\_oroi < r$$
$$EVENT = 0, \quad Otherwise. \quad (2)$$

$prct\_iroi$ and $prct\_oroi$ refer to a certain motion percentile in the region of interest and outside the region of interest, respectively. $EVENT = 1$ means the corresponding epoch is detected as being an event by event detection unit 40 and $EVENT = 0$ means the epoch is detected as being not an event. An example of event detection results is illustrated and discussed with reference to Fig. 4 below.

Baby in bed determination unit 60 is configured to, based on events detected by event detection unit 40 and based on motion data from inside the region of interest 22, 28, whether the baby 7 is in bed 5 or not. In this example, as long as events are detected, motion data from within the region of interest is analyzed and rules are applied to identify the baby 7 in/out of bed 5 status. Preferably, the original epoch based motions, without filtering out the small motions by filter unit 50, are used, while also motion data which has not been segmented into epochs can be used in other examples.

According to a first rule, if a maximum motion amplitude of motion data in the region of interest 22, 28 of all events, i.e. of all epochs of the motion data during which events are determined, over the entire recording, for instance, the 24hrs exemplarily described above, are too small, the motions are likely to be noises or other small disturbances. Accordingly, in case the maximum motion amplitude is less than a globally defined threshold $thr\_global$, the status of the baby 7 is determined to be out of bed throughout the entire recording.

$$Status = OUT \ for \ the \ entire \ recording, if \max(mot\_iroi \ |EVENT = 1| < thr\_global \quad (3)$$

The second rule is applied in case the maximum motion amplitude within the region of interest exceeds the global threshold only. In this case, the in/out of bed status is determined between each two events, which can be directly consecutive events or two events having intermediate events in between, by analyzing whether sufficient motions caused by body movements of baby 7, for instance during sleep, are present. As an example, a certain period between two events can be comprised of a total $k$ epochs. In this example, preferably only the epochs in the middle of the period are taken into account for determining the status of baby 7 in/out of bed 5 and epochs that are too close to the events defining the beginning and the end of the period are skipped. Motions from those epochs are advantageously skipped since they are likely due to activities of the parents or other objects rather than body movements of baby 7, since the putting baby to bed and taking baby out of bed activities often last for a few minutes, i.e. a duration exceeding the duration of a single epoch in which the event is detected. In one example, a percentage $p$ of epochs both at the beginning and the end of the period are excluded, while the percentage can of course also be different for the beginning and the end of the period.

The number of epochs $k$ is thus reduced to, for instance, $k(1-2p)$. A percentage of non-zero epochs, i.e. epochs with motion out of the epochs within the period, can then be determined by counting the number of non-zero epochs and dividing them by the number of epochs of the period reduced by the skipped epochs.

Finally, the whole period is then identified as "baby in bed" if the percentage of non-zero epochs $p\_mot$ is larger than a local threshold $thr\_local$ and as "baby out of bed" otherwise. The output of baby in bed determination unit 60 will also exemplarily be described with reference to Fig. 4 below.

Finally, smoothing unit 70 is configured to smooth the determination result of baby in bed determination unit 60. Smoothing unit 70 thereby provides a form of post-processing the determination result. While baby in bed determination unit 60 can provide a determination, in which the status of the baby would change over very short periods of time, for instance one or two epochs, which do not or very unrealistically happen in real life. Smoothing unit 70 then smoothes the detection result for the purpose of filtering out these very short periods. For example, a median filter with a sliding window of particular window size can be used, wherein also other suitable units for filtering out or smoothing those short periods can of course be used.

Fig. 4 exemplarily and schematically summarizes the output of the various units of system 1 for monitoring a baby for the exemplary stream of video images 200 referred to already in Fig. 2.

All six plots 410, 420, 430, 440, 450 and 460 are illustrated for the same period of time in terms of 30s epoch on the horizontal axis.

A first plot 410 illustrates a ground-truth of baby in bed 412 and baby out of bed 414, which has been annotated from the video recording.

Plots 420 and 430 illustrate a magnitude of motion data 14 for customized region of interest 28 illustrated in Fig. 3B. In plot 420, motion from inside the customized region of interest 28 is illustrated, while plot 430 illustrates the corresponding motion outside the region of interest.

Based on plots 420 and 430, events detected by event detection unit 40 are illustrated in plot 440.

Based on the events illustrated in plot 440, the status whether baby 7 is in bed or not as determined by baby in bed determination unit 60 is illustrated in plot 450. It can be seen that baby in bed 452 and baby out of bed 454 already is in good accordance to the results of the ground-truth plot 410, while short periods 456 which are unlikely to happen in real life occur at several occasions during the recording.

Finally, based on the status determined by baby in bed determination unit 60 is smoothed by smoothing unit 70 and the output of smoothing unit 70 is illustrated as plot 460. The very short periods still visible in plot 450 are smoothed out and the determined status of baby in bed 462 and baby out of bed 464 very accurately corresponds to the ground-truth plot 410.

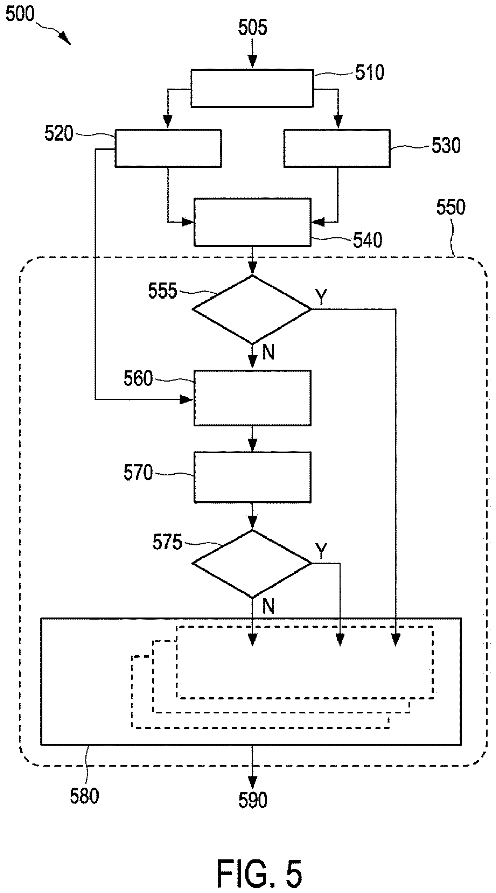

Fig. 5 schematically and exemplarily illustrates a flow of a baby in/out of bed detection method 500. All the parameters described with respect to the examples of Fig. 1 to 4 ($prct\_iroi$, $prct\_oroi$, $r$, $thr\_global$, $p$, $thr\_local$) can be preferably be optimized to maximize the accuracy of the in and out of bed detection.

In step 510, video frames 505 including a region of interest configuration are input to carry out motion estimation for within the region of interest and outside the region of interest. The region of interest can for instance comprise at least one of a fixed region of interest 22 and a customized region of interest 28.

In step 520, the motion inside the region of interest is summed for epochs of 30s, in step 530 the motion outside the region of interest is accordingly summed for the same epoch of 30s each.

Based on the motions inside and outside the region of interest determined in steps 520 and 530, events are detected in step 540.

Subsequently, based on events determined in step 540 and motion data from within the region of interest, frame 550 illustrates the subset of method 500 which provides for the in and out of bed detection.

In step 555, it is decided based on the events detected in step 540, whether a maximum of the motion amplitude of the motion within the region of interest remains below a global threshold $thr\_global$. In the affirmative, the baby is determined to be out of bed for the entire recording, which is forwarded to a post-processing step 580.

In the negative, the method proceeds with a step 560, in which motion within the region of interest between two events is found and motions which are too close to the time of the events is removed.

In step 570, a percentage of non-zero epochs $p\_mot$ is computed.

Next, in step 575 it is decided whether the percentage of non-zero epochs is smaller than or equal to a local threshold $thr\_local$. In the affirmative, the baby is determined to be out of bed for the period, wherein in the negative, it is determined that the baby is in bed for the period.

In step 580, post-processing over a window is carried out so that, among others, very short periods of baby in bed/baby out of bed can be filtered out.

Eventually, at step 590 method 500 outputs the baby in/out of bed status.

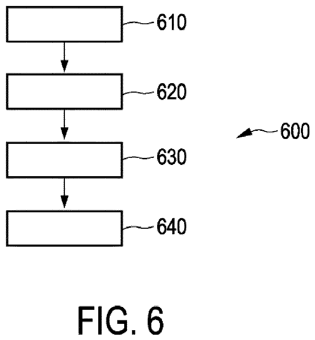

A further example of a method 600 of monitoring a baby is schematically illustrated with reference to Fig. 6.

In step 610, motion data indicative of motions within a stream of video images is provided.

In step 620, a region of interest in the video images is provided.

In step 630, the provided motion data is classified into at least motion data from inside the region of interest and from outside the region of interest.

In step 640, an event is detected by evaluating motion data from inside the region of interest and motion from outside the region of interest.

It should be noted that steps of methods 500 and 600 can be combined among each other as well as with steps exemplarily carried out by units of system 1 as described with reference to Figs. 1 to 4.

The validity of this invention was examined by analyzing 77 recordings of approximately 24 hrs each from five babies which were all less than two years old. No requirement regarding camera placement except for ensuring the view of the baby was in force. Two configurations of a fixed region of interest with 90% of the recorded video area in the middle and a customized region of interest including the bed frame only are compared, wherein parameters in the detection model are optimized separately for the two configurations. While overall accuracy exceeds 95% in both configurations, the customized region of interest configuration performs, as expected, even better at about two percentage points than the fixed region of interest configuration.

System 1 for monitoring a baby can in one example used for monitoring devices or situations where motions are used or can be estimated, for instance to detect in/out of bed status for variable sensors, radar sensors and LED sensors. In this example, system 1 can communicate with such sensors and provide the determined in/out of bed status of baby 7 to the respective sensor.

While the region of interest configuration has been exemplarily described as a non-overlapping configuration which is either fixed or customized, it can also have an overlapping region including motions from both parents and baby in other examples. Further, as indicated above, the region of interest can be in any percentages of the video frames, of any shape and also multiple regions of interest areas at any shapes can be defined in the video frames. Additionally, the region of interest can be fixed, e.g. region of interest 22, and can also be customized, e.g. region of interest 28, where the customization can be done by users manually or by a dedicated algorithm automatically.

Figs. 7A and 7B show schematically and exemplarily motion patterns for determining misalignment of the region of interest 22, 28. Misalignment of the region of interest 22, 28 can cause incorrect determination of the baby being in bed or not. There are several occurrences which can cause misalignment of the region of interest 22, 28.

First of all, motion of the camera 2 itself can misalign the region of interest 22, 28, for instance such that bed 5 of baby 7 is partly or completely outside the field of view of the camera 2. Such motion can either be unintentional, for instance through moving or picking up camera 2 when cleaning and dusting the area, or intentional, for instance to use functions of camera 2.

Next, a misalignment of the region of interest 22, 28 can result from a change of the layout of the room which the camera monitors, ranging from only changing a position of bed 5, changing the position of the camera and changing the complete layout including the position of the camera.

Figs. 7A and 7B show two motion patterns 700 and 710 indicating a direction in which each of the pixels of a video image of the camera moves. Motion pattern 700 shows different pixels moving in different directions, which is an indication that some object in the field of view moves. In the example, an object is moving away from the camera and downwards. Motion pattern 710 shows that all pixels simultaneously move in the same direction. This indicates a motion of the camera itself and can be used, for instance, to detect intentional and unintentional motion of the camera itself.

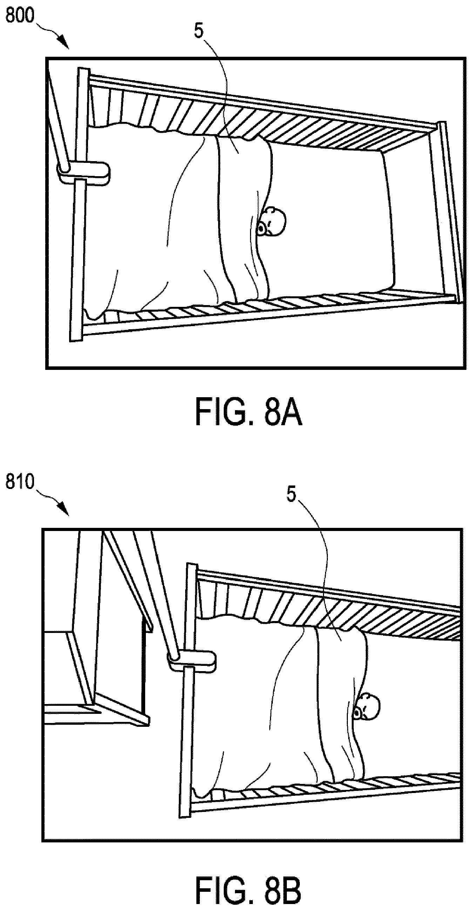

Figs. 8A and 8B schematically and exemplarily illustrate two camera views 800, 810. The camera views are, for instance, a standard RGB view as captured by camera 2. View 800 covers bed 5 entirely, while bed 5 is moved in view 810 so that one edge of bed 5 lies outside view 810. Preferentially, upon installation of the system 1 when camera 2 is activated and no motion is detected, a picture, e.g. view 800, is taken, and boundaries, such as outlines of bed 5, are easily detectable. When camera 2 is intentionally or unintentionally moved, the alignment of these boundaries will change, wherein this change can be detected. Optionally thresholds can be used to define what amount of movement is acceptable and what is not. Further optionally and preferably, different ways of visualization can be employed to highlight outlines like the edges of bed 5, such as contrast view. Using, for example contrast view, it can be easily determined using image analysis that the outlines of bed 5 are no longer completely visualized, for instance.

Additionally or alternatively to the motion and image data analysis described above, other methods can be employed to configure region of interest providing unit 20 to determine a misalignment of the region of interest 22, 28.

First, additional sensors can be provided in connection with camera 2 and/or bed 5 which provide information indicative of a motion of camera 2 and/or bed 5. Without being limited, one or more of accelerometers, gyroscopes and proximity sensors can be used. If provided together with camera 2, a change on the planar axes and/or the tilt angle of the accelerometer indicate a movement of camera 2 and thus a change in the view of camera 2. If motion reaches a certain threshold, it is likely the view has significantly changed. Additionally or alternatively, a gyroscope is able to detect a change in the viewing angle and thus the view of camera 2. Proximity sensors, which include radar, acoustic sonar, ultrasound, can detect both a change of position of camera 2 relative to the room and a change of items in the room, such as bed 5.

Finally, one or more markers can be added, for instance supplied with camera 2, which are to be attached to an object of interest within the region of interest 22, 28, preferably to bed 5.

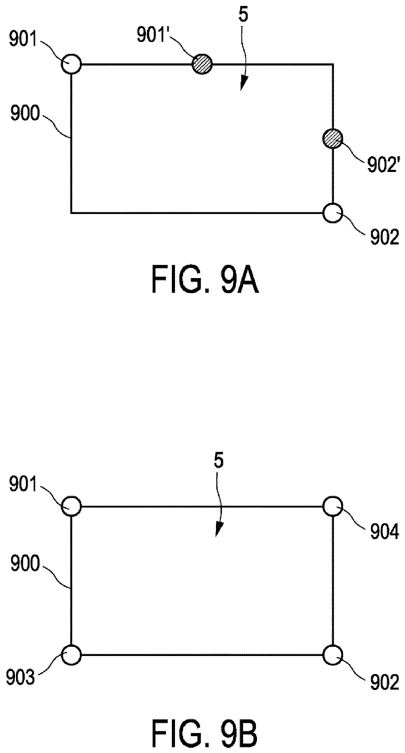

Fig. 9A and 9B show different arrangements for two markers 901, 902 and four markers 901, 902, 903, 904, respectively, while also only one marker or a different number can likewise be employed. Fig. 9A shows markers 901, 902 placed on the crossing of two edges 900 of, for instance, bed 5 and markers 901', 902' placed in the middle of two edges. Fig. 9B shows four markers 901, 902, 903, 904 placed. Figs. 9A and 9B only show the edges 900 of bed 5 for simplicity of illustration. The two or more markers 901-904 can thus outline edges of bed 5 and allow a facilitated detection of the region of interest.

The region of interest providing unit 20 can measure distances between the markers 901-904 and determinate a visibility of the markers 901-904. Preferably, a feedback can be given to the user by system 1 based on these measured distances and/or determined visibilities. The feedback given to the user can for instance be to order manual adjustment of the position of camera 2, so that the user brings markers 901-904 back into their desire positions.

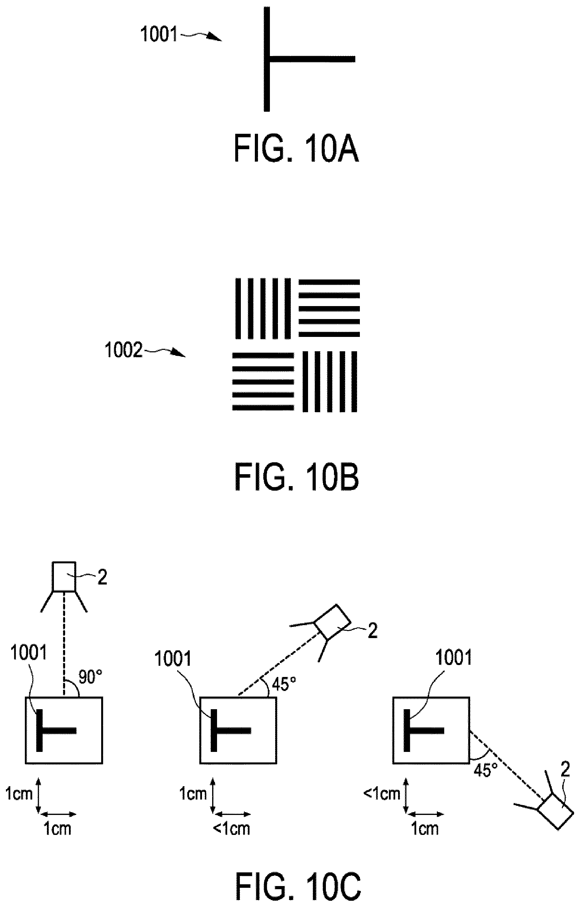

In other examples, also a single marker using a pattern can be used to detect if the view of camera has changed over time, which will be described with reference to Figs. 10A to 10C. The pattern can have a known size and therefore be used to determine a change in the distance of bed 5 in relation to camera 2, thus providing input to calculate a new dimension of the region of interest. Further, it can be used to determine a change in mounting angle of the camera 2, thereby providing input to calculate the deformation of bed 5 in respect to the region of interest 22, 28.

Figs. 10A and 10B show two further examples of markers 1001 and 1002, which show a respective pattern each. The dimensions of the lines of the pattern of the markers 1001 and 1002 as well as the overall dimension are known. Both patterns of marker 1001 and 1002 have as least one pair of perpendicular lines, which facilitates calculation of distances and orientations of the spot to which markers 1001 or 1002 are attached.

Fig. 10C illustrates, taking the example or marker 1001, how the two stripes or edges of the T-shaped pattern of marker 1001 appear shorter depending on the viewing position of camera 2, such that a distance and orientation can with respect to camera 2 can be determined. In the first situation illustrated in Fig. 10C, the position of camera 2 is perfectly perpendicular to marker 1001 and both stripes appear of the same length, for instance 1 cm. Based on the dimensions of marker 1001 on the recorded camera image and the known extension of marker 1001 in real space, a distance between marker 1001 and camera 2 can preferably be triangulated. In the second and third situation, camera 2 is moved under 45° with respect to the first and the second stripe, respectively. It can be seen that the stripe with respect to which camera 2 was moved appears optically shorter, while the stripe which is perpendicular is still of the original length.

The patterns of markers 1001 and 1002 are of course just examples and other suitable patterns can be used instead.

Any of markers 901-904, 1001, 1002 can be an infrared reflector which is transparent to the human eye and reflects wavelengths in the infrared region. Thereby, no visually bothering markers 901-904, 1001, 1002 have to be provided.

Since the baby in/out of bed status is an important basic information about baby sleep and development, information provided by system 1 can advantageously be used to assist in evaluating baby's sleep, including sleep and wake classification, sleep staging, sleep quality assessment, crying analysis and so on, to analyze long term baby sleep and activity rhythm, to help coach parents and reassurance, and the like.

A computer program may be stored/distributed on a suitable medium, such as an optical storage medium or a solid-state medium, supplied together with or as part of other hardware, but may also be distributed in other forms, such as via the Internet or other wired or wireless telecommunication systems including being downloadable or purchasable via an app store.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

**Claims**

1. A system for monitoring a baby (7), comprising
   a motion data providing unit (10) for providing motion data (12, 14) indicative of motions within a stream of video images (200),
   a region of interest providing unit (20) for providing a region of interest (22) in the video images (200),
   a classifying unit (30) for classifying the provided motion data (12, 14) into at least motion data (12, 14) from inside the region of interest (22, 28) and from outside the region of interest (22, 28),
   an event detection unit (40) for detecting an event by evaluating motion data (12, 14) from inside the region of interest (22, 28) and motion data (12, 14) from outside the region of interest (22, 28).

2. The system according to claim 1, wherein at least one of the motion data (12, 14) from inside the region of interest (22, 28) and the motion data (12, 14) from outside the region of interest (22, 28) is provided as the sum of motion over a period of time of the stream of video images (200).

3. The system according to claim 1, wherein the event detection unit (40) is configured to detect at least one of i) baby (7) put to bed (5) and ii) baby (7) taken out of bed (5) as an event.

4. The system according to claim 1, further comprising a filter unit (50) for filtering motion data (12, 14) from inside the region of interest (22, 28) and from outside the region of interest (22, 28).

5. The system according to claim 1, wherein the event detection unit (40) is configured to detect an event if i) motions inside the region of interest (22, 28) and motions outside the region of interest (22, 28) are larger than zero and ii) a ratio between motions inside the region of interest (22, 28) and motions outside the region of interest (22, 28) are below a threshold.

6. The system according to claim 1, further comprising a baby in bed determination unit (60) for determining whether the baby (7) is in bed (5) or not based on events detected by the event detection unit (40) and based on motion data from inside the region of interest (22, 28).

7. The system according to claim 6, wherein the baby in bed determination unit (60) is configured to compare an amplitude of motion data from inside the region of interest (22, 28) with a predetermined threshold and to determine the baby (7) as out of bed (5) for the entire time of the stream of video images (200) in case the amplitude does not exceed the predetermined threshold.

8. The system according to claim 6, wherein the baby in bed determination unit (60) is configured to define a period between two events and to analyze the percentage of time with motion during the period for determining whether the baby (7) is in bed (5) or not.

9. The system according to claim 8, wherein the baby in bed determination unit (60) is configured to discard a predetermined percentage of the period between two events from the motion data, wherein the motion data is discarded at the beginning and the end of the period and to determine the baby (7) as in bed (5) for the period in case the percentage of time with motion exceeds a predetermined threshold.

10. The system according to claim 1, wherein the region of interest providing unit (20) is configured to provide a customized region of interest (28) based on the video images (200), wherein the customization is either determined automatically based on previous video images (200) or manually selected by a user.

11. The system according to claim 1, wherein the region of interest providing unit (20) is configured to detect a misalignment of the region of interest and to at least one of i) notify about a detected misalignment and ii) correct the region of interest.

12. The system according to claim 11, wherein
the region of interest providing unit (20) is configured to detect the misalignment of the region of interest based on motion of a camera (2) which records the stream of video images (200) or a change of view of the camera (2) based on at least one of the provided motion data and the video images, wherein
the region of interest providing unit (20) is configured to determine motion of the camera (2) or a change of view of the camera (2) by at least one of i) analyzing the uniformity of motion over the video images and ii) determining and monitoring outlines of a bed (5) of the baby (7) in the video images.

13. The system according to claim 11, wherein
the region of interest providing unit (20) is configured to detect a misalignment of the region of interest (22, 28) based on data from a sensor component, wherein
the sensor component includes at least one of an accelerometer, a gyroscope and a proximity sensor, or wherein the region of interest providing unit (20) is configured to detect a misalignment of the region of interest (22, 28) based on one or more markers (901, 901', 902, 902', 903, 904, 1001, 1002), preferably infrared reflective markers, attached to an object, preferably a bed (5) of a baby (7), within the region of interest (22, 28).

14. A method of monitoring a baby (7), comprising
providing (610) motion data (12, 14) indicative of motions within a stream of video images (200),
providing (620) a region of interest (22, 28) in the video images,
classifying (630) the provided motion data (12, 14) into at least motion data (12, 14) from inside the region of interest (22, 28) and from outside the region of interest (22, 28),
detecting (640) an event by evaluating motion data (12, 14) from inside the region of interest (22, 28) and motion data (12, 14) from outside the region of interest (22, 28).

15. A computer program for monitoring a baby (7), the computer program comprising program code means for causing a system (1) as defined in claim 1 to carry out the method (100) as defined in claim 14, when the computer program is run on the system (1).

10

20

30

40

1

50

60

70

5

7

2

25

27

# FIG. 1

FIG. 2A

FIG. 2B                    FIG. 2C

FIG. 3A

FIG. 3B

FIG. 4

FIG. 5

FIG. 6

FIG. 7A

FIG. 7B

800

5

FIG. 8A

810

5

FIG. 8B

FIG. 9A

FIG. 9B

FIG. 10A

FIG. 10B

FIG. 10C

Europäisches
Patentamt

European
Patent Office

Office européen
des brevets

# EUROPEAN SEARCH REPORT

Application Number

EP 17 16 7955

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 023 957 A1 (PANASONIC IP MAN CO LTD [JP]) 25 May 2016 (2016-05-25) * abstract * * figures 8A-8C, 10 * * paragraphs [0050] - [0079] * ----- | 1-15 | INV. H04N7/18 G06K9/00 A61B5/00 |
| A,D | WO 2015/091582 A1 (KONINKL PHILIPS NV [NL]) 25 June 2015 (2015-06-25) * abstract * * page 7, line 1 - page 9, paragraph 16 * ----- | 1-15 | |

TECHNICAL FIELDS
SEARCHED (IPC)

H04N
A61B
G06K

The present search report has been drawn up for all claims

| Place of search | Date of completion of the search | Examiner |
|---|---|---|
| The Hague | 3 October 2017 | Naci, Suphi Umut |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 396 946 A1**

## ANNEX TO THE EUROPEAN SEARCH REPORT
## ON EUROPEAN PATENT APPLICATION NO.

EP 17 16 7955

03-10-2017

| Patent document cited in search report | | Publication date | Patent family member(s) | | Publication date |
|---|---|---|---|---|---|
| EP 3023957 | A1 | 25-05-2016 | EP | 3023957 A1 | 25-05-2016 |
| | | | JP | 5866551 B1 | 17-02-2016 |
| | | | JP | 2016099790 A | 30-05-2016 |
| | | | US | 2016148493 A1 | 26-05-2016 |
| | | | WO | 2016079888 A1 | 26-05-2016 |
| WO 2015091582 | A1 | 25-06-2015 | CN | 106028915 A | 12-10-2016 |
| | | | EP | 3082572 A1 | 26-10-2016 |
| | | | JP | 2017503566 A | 02-02-2017 |
| | | | US | 2016310067 A1 | 27-10-2016 |
| | | | WO | 2015091582 A1 | 25-06-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015091582 A1

- WO 2015091582 A